# EUROPEAN PATENT APPLICATION

(11) **EP 4 113 125 A1**
(43) Date of publication of application: **04.01.2023**
(21) Application number: 21759718.6
(22) Date of filing: 03.02.2021
(51) Int. Cl.: G01N 37/00, G01N 35/08, G01N 27/00

(54) **SENSOR PACKAGE AND SENSOR MODULE**

(30) Priority: 28.02.2020 JP 2020033843
(71) Applicant: Kyocera Corporation, Kyoto-shi Kyoto 612-8501 (JP)
(72) Inventor: TAJIMA Masahiko, Kyoto-shi, Kyoto 612-8501 (JP); Hisashi Sakai, Kyoto-shi, Kyoto 612-8501 (JP); Maehara Tadatomo, Kyoto-shi, Kyoto 612-8501 (JP)
(74) Representative: TBK
(86) International application number: PCT/JP2021/003976
(87) International publication number: WO 2021/171940

(57) **Abstract**

A sensor package 10 includes a container 18 and a plurality of sensors 19. The container 18 includes inside an internal flow path 21 allowing a fluid to flow in a first direction d1 having linearity. The plurality of sensors 19 is located in the internal flow path 21and arrayed in the first direction d1. The sensors 19 detect a component to be detected within the fluid.

## Description

### CROSS-REFERENCE TO RELATED APPLICATIONS

This application claims priority of Japanese Patent Application No. 2020-33843 filed in Japan on February 28, 2020 and the entire disclosure of this application is hereby incorporated by reference.

### TECHNICAL FIELD

The present invention relates to a sensor package and a sensor module.

### BACKGROUND OF INVENTION

A known measurement device includes a quartz vibrator functioning as a sensor disposed inside a flow path tube and detecting an odor in a space (refer to Patent Literature 1). An odor is perceived by an organism from a single molecule or a group of molecules made up of a plurality of different molecules and a known technology uses a plurality of sensors to detect odors (refer to Patent Literature 2).

### CITATION LIST

### PATENT LITERATURE

Patent Literature 1: Japanese Unexamined Patent Application Publication No. 2012-2691
Patent Literature 1: International Publication No. 2018/211642

### SUMMARY

In a first aspect, a sensor package includes a container and a plurality of sensors. The container includes inside an internal flow path allowing a fluid to flow in a first direction having linearity. The plurality of sensors is located in the internal flow path and arrayed in the first direction. The plurality of sensors is configured to detect a component to be detected within the fluid.

In a second aspect, a sensor module includes a switching unit, a sensor package, and a pump unit. The switching unit is disposed downstream of a first flow path and a second flow path and configured to selectively switch open/closed states of the first flow path and the second flow path. The sensor package is disposed downstream of the switching unit and includes a container and a plurality of sensors. The container includes inside an internal flow path allowing a fluid to flow in a first direction having linearity. The plurality of sensors is located in the internal flow path in the first direction and configured to detect a component to be detected within the fluid. The pump unit is disposed downstream of the sensor unit and configured to draw a fluid downstream.

### BRIEF DESCRIPTION OF THE DRAWINGS

FIG. 1 is a schematic diagram of a sensor module according to an embodiment.
FIG. 2 is a perspective view illustrating a cross section of a sensor package in FIG. 1 taken along a plane perpendicular to a second direction.
FIG. 3 is a perspective view illustrating an internal flow path in FIG. 2.
FIG. 4 is a sectional view of a main part in FIG. 2 taken along a plane perpendicular to a first direction.
FIG. 5 is a see-through view illustrating the internal flow path in FIG. 2 in which the sensor package is viewed in a direction normal to a bottom surface of the sensor package.
FIG. 6 is a perspective view illustrating a variation of the internal flow path in FIG. 2.
FIG. 7 is a perspective view illustrating the exterior of the sensor in FIG. 2.
FIG. 8 is a sectional view of the sensor module in FIG. 2 taken along a plane perpendicular to the first direction.
FIG. 9 is a functional block diagram illustrating the schematic configuration of the sensor module in FIG. 1.
FIG. 10 is a diagram schematically illustrating an example of fluid flow.
FIG. 11 is a diagram schematically illustrating an example of fluid flow.
FIG. 12 is a see-through view for explaining the size of an internal flow path of Example 1.
FIG. 13 is a sectional view for explaining the size of an internal flow path of Example 1.
FIG. 14 is a distribution diagram of residence time at each position in the internal flow path in Example 1.
FIG. 15 is a diagram of the flow velocity distribution for the internal flow path in Example 1 at a central position and at positions overlapping detection units in the second direction and at positions overlapping the detection units in the first direction.
FIG. 16 is a diagram of the pressure distribution in the internal flow path in Example 1 at a central position and at positions overlapping detection units in the second direction and at positions overlapping the detection units in the first direction.
FIG. 17 is a graph illustrating the ratio of gas with respect to elapsed time from the start of inflow for detection units arrayed sequentially from the inflow side to the outflow side of the fluid in the internal flow path in Example 1.
FIG. 18 is a table showing arrival times of a gas at detection units arrayed sequentially from the inflow side to the outflow side of the fluid in internal flow paths of Examples and a comparative example.
FIG. 19 is a distribution diagram of residence time at each position in an internal flow path in Example 2.
FIG. 20 is a diagram of the flow velocity distribution in the internal flow path in Example 2 at a central position and at positions overlapping detection units in the second direction and at positions overlapping the detection units in the first direction.
FIG. 21 is a diagram of the pressure distribution in the internal flow path in Example 2 at a central position and at positions overlapping detection units in the second direction and at positions overlapping the detection units in the first direction.
FIG. 22 is a graph illustrating the ratio of gas with respect to elapsed time from the start of inflow for detection units arrayed sequentially from the inflow side to the outflow side of the fluid in the internal flow path in Example 2.
FIG. 23 is a distribution diagram of residence time at each position in an internal flow path in Example 3.
FIG. 24 is a diagram of the flow velocity distribution in the internal flow path in Example 3 at a central position and at positions overlapping detection units in the second direction and at positions overlapping the detection units in the first direction.
FIG. 25 is a diagram of the pressure distribution in the internal flow path in Example 3 at a central position and at positions overlapping detection units in the second direction and at positions overlapping the detection units in the first direction.
FIG. 26 is a graph illustrating the ratio of gas with respect to elapsed time from the start of inflow for detection units arrayed sequentially from the inflow side to the outflow side of the fluid in the internal flow path in Example 3.
FIG. 27 is a diagram illustrating the conceptual structure of a flow path of Comparative Example 1.
FIG. 28 is an external view of a first c-shaped connecting tube in FIG. 27.
FIG. 29 is an external view of a second c-shaped connecting tube in FIG. 27.
FIG. 30 is an external view of a z-shaped connecting tube in FIG. 27.
FIG. 31 is an external view of an l-shaped connecting tube in FIG. 27.
FIG. 32 is an external view of a curved tube in FIG. 27.
FIG. 33 is a distribution diagram of residence time at each position in the flow path in Comparative Example 1.
FIG. 34 is a diagram of the flow velocity distribution at each position in the flow path in Comparative Example 1.
FIG. 35 is a pressure distribution diagram at each position in the flow path in Comparative Example 1.
FIG. 36 is a conceptual diagram illustrating the positions of detection units in Comparative Example 1.
FIG. 37 is a graph illustrating the ratio of gas with respect to elapsed time from the start of inflow for detection units arrayed sequentially from the inflow side to the outflow side of the fluid in the internal flow path in Comparative Example 1.

### DESCRIPTION OF EMBODIMENTS

Hereafter, embodiments according to the present disclosure are described in detail with reference to the drawings.

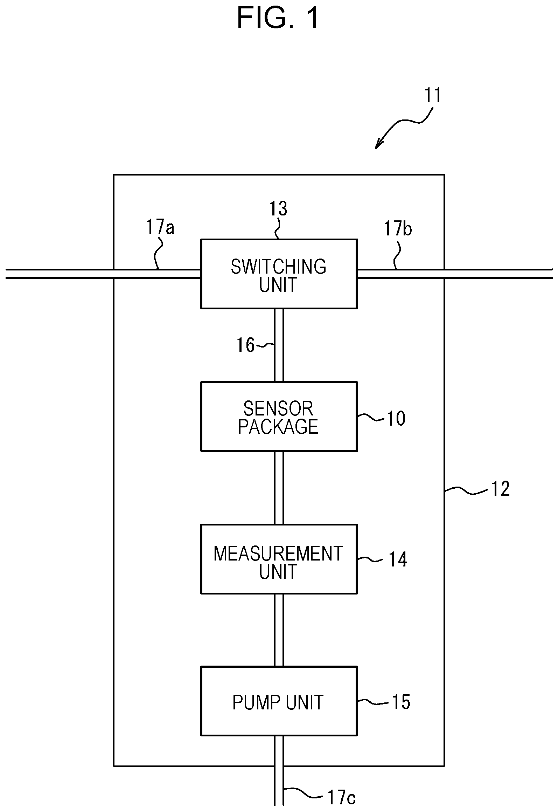

FIG. 1 is a schematic diagram of a sensor module 11 including a sensor package 10 according to an embodiment of the present disclosure. The sensor module 11 includes, for example, a housing 12. Various functional units of the sensor module 11 are housed inside the housing 12. A fluid is supplied to the sensor module 11. The sensor module 11 can calculate the concentration of a first component, which is a component to be detected contained in a test fluid, based on a fluid to be tested (test fluid) and a fluid to be compared (control fluid). In this specification, hereinafter, the side from which a fluid is supplied is also referred to as an upstream side and a side from which the fluid is discharged is also referred to as a downstream side.

The sensor module 11 includes, inside the housing 12, a switching unit 13, the sensor package 10, a measurement unit 14, and a pump unit 15. In the sensor module 11, the switching unit 13, the sensor package 10, the measurement unit 14, and the pump unit 15 are disposed in this order from the upstream side along a single flow path 16. The flow path 16 is, for example, a tube-shaped member such as a tube. A first flow path 17a and a second flow path 17b are further connected to the switching unit 13 on the upstream side. A fluid is supplied to the inside of the sensor module 11 from the first flow path 17a and the second flow path 17b and the fluid is discharged to outside the sensor module 11 from a third flow path 17c connected to the downstream side of the pump unit 15.

A test fluid is supplied to the first flow path 17a. A control fluid is supplied to the second flow path 17b. A discharge fluid is discharged to the third flow path 17c. The first flow path 17a, the second flow path 17b, and the third flow path 17c are, for example, tube-shaped members such as tubes.

The switching unit 13 selectively switches the open/closed states of the first flow path 17a and the second flow path 17b. In other words, the switching unit 13 can selectively connect either one of the first flow path 17a and the second flow path 17b to the flow path 16. Therefore, when the first flow path 17a is connected to the flow path 16 by the switching unit 13, the second flow path 17b is not connected to the flow path 16. In this case, the test fluid is supplied to the flow path 16 via the first flow path 17a. On the other hand, when the second flow path 17b is connected to the flow path 16 by the switching unit 13, the first flow path 17a is not connected to the flow path 16. In this case, the control fluid is supplied to the flow path 16 via the second flow path 17b. The switching unit 13 may include, for example, a valve capable of switching to the first flow path 17a or the second flow path 17b.

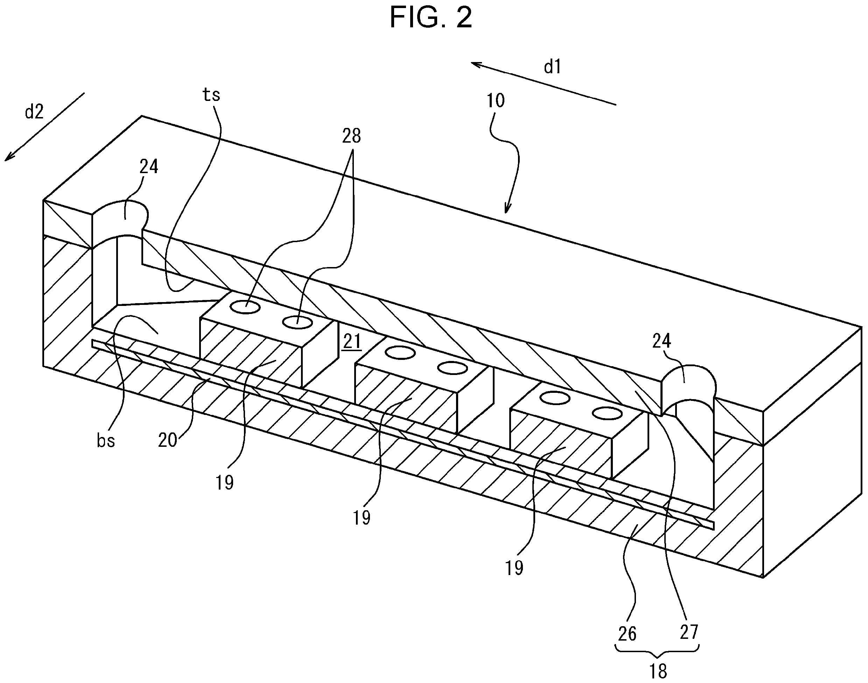

As illustrated in FIG. 2, the sensor package 10 includes a container 18 and a plurality of sensors 19. The sensor package 10 may further include a heater 20.

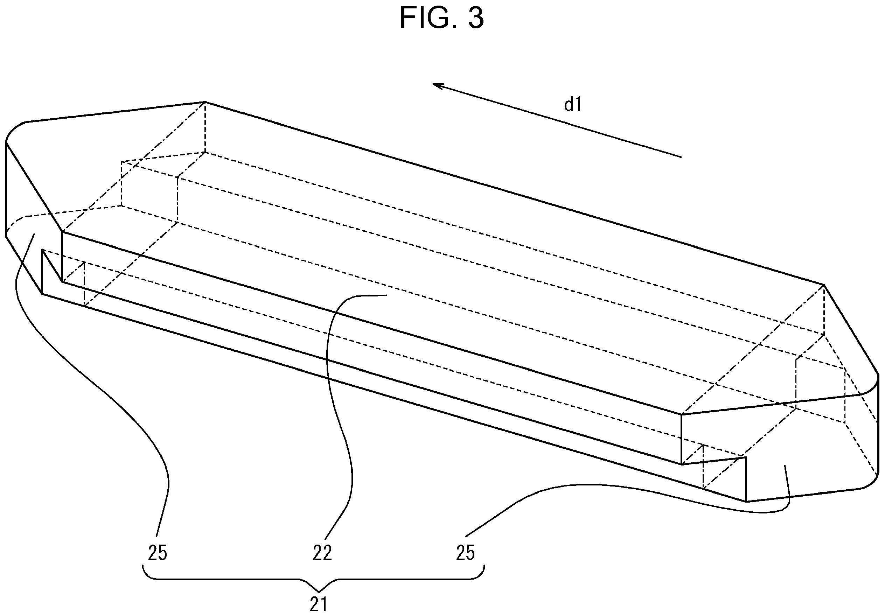

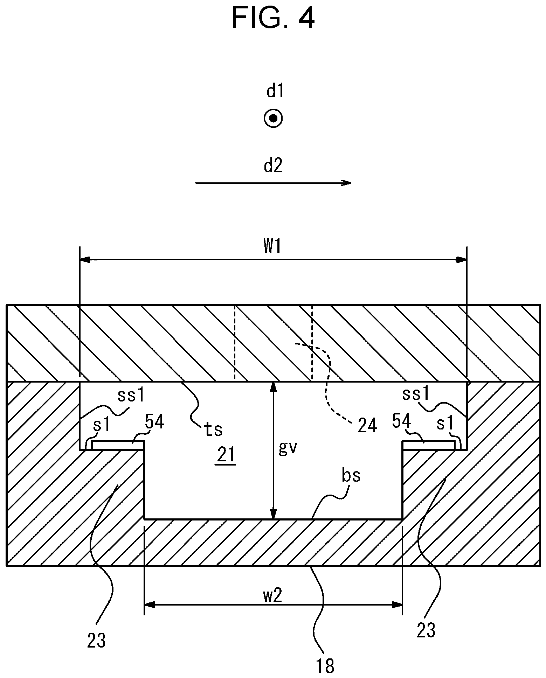

The container 18 includes inside an internal flow path 21. The internal flow path 21 allows a fluid to flow in a first direction d1 having linearity. As illustrated in FIG. 3, the internal flow path 21 may include, for example, a main part 22 defined by a cylindrical inner wall extending in the first direction d1. As illustrated in FIG. 4, the internal flow path 21 may be, for example, partially defined by a flat bottom surface bs. The internal flow path 21 may be, for example, partially defined by a flat top surface ts facing the bottom surface bs.

A spacing gv between the bottom surface bs and the top surface ts may be from 1.5 times to 3 times the height of the sensors 19, which are described later. When the spacing gv is 1.5 times or more, a sufficient space is ensured for a fluid to flow. When the spacing gv is 1.5 times or more, the pressure distribution becomes uniform and the outputs of the sensors 19 are stabilized. When the spacing gv is 3 times or less, the sensor package 10 can avoid becoming unnecessarily large. When the spacing gv is 3 times or less, a reduction in the flow velocity can be suppressed. In this embodiment, the spacing gv between the bottom surface bs and the top surface ts is twice the height of the sensors 19. Therefore, in this embodiment, the spacing between the top surface ts and each sensor 19 fixed to the bottom surface bs is the same as the height of each sensor 19.

The main part 22 may be partially defined by side surfaces ss1 that are perpendicular to the bottom surface bs and parallel to the first direction d1. The side surfaces ss1 may be connected to the bottom surface bs at both ends of the bottom surface bs in a second direction d2, which is parallel to the bottom surface bs and perpendicular to the first direction d1. The side surfaces ss1 may be connected to the top surface ts at both ends of the top surface ts in the second direction d2. The spacing between the two side surfaces ss1, i.e., a width w1 of the internal flow path 21 in the second direction d2 may be from 1.5 times to 3 times the width of each sensor 19 described later. When the width w1 is 1.5 times or more, a sufficient space is ensured for a fluid to flow. When the width w1 is 1.5 times or more, the pressure distribution becomes uniform and the outputs of the sensors 19 are stabilized. A reduction in the flow velocity can be suppressed. When the width w1 is 3 times or less, the sensor package 10 can avoid becoming unnecessarily large. When the width w1 is 3 times or less, a reduction in the flow velocity can be suppressed. In this embodiment, the width w1 of the internal flow path 21 is twice the width of the sensors 19.

A step portion 23 extending in the first direction d1 may be formed on at least one of the side surfaces ss1 of the main part 22. In this embodiment, the step portions 23 are formed on both the side surfaces ss1. Step portion electrodes 53 for electrically connecting to the sensors 19 may be provided on surfaces s1 of the step portions 23 that face the top surface ts. The height of the step portions 23 from the bottom surface bs may be greater than or equal to the height of the sensors 19, which are described later. A width w2 in the second direction d2 between the step portions 23 formed on the two side surfaces ss1 may be from 1.1 times to 1.5 times the width of the sensors 19, which are described later. When the width w2 is 1.1 times or more, a sufficient space is ensured for a fluid to flow. When the width w2 is 1.1 times or more, the pressure distribution becomes uniform and the outputs of the sensors 19 are stabilized. When the width w2 is 1.5 times or less, the sensor package 10 can avoid becoming unnecessarily large. Surfaces of the sensors 19 and the step portions 23 facing the top surface ts are contiguous with each other in the second direction d2, thereby ensuring a space for suppressing a reduction in the flow velocity.

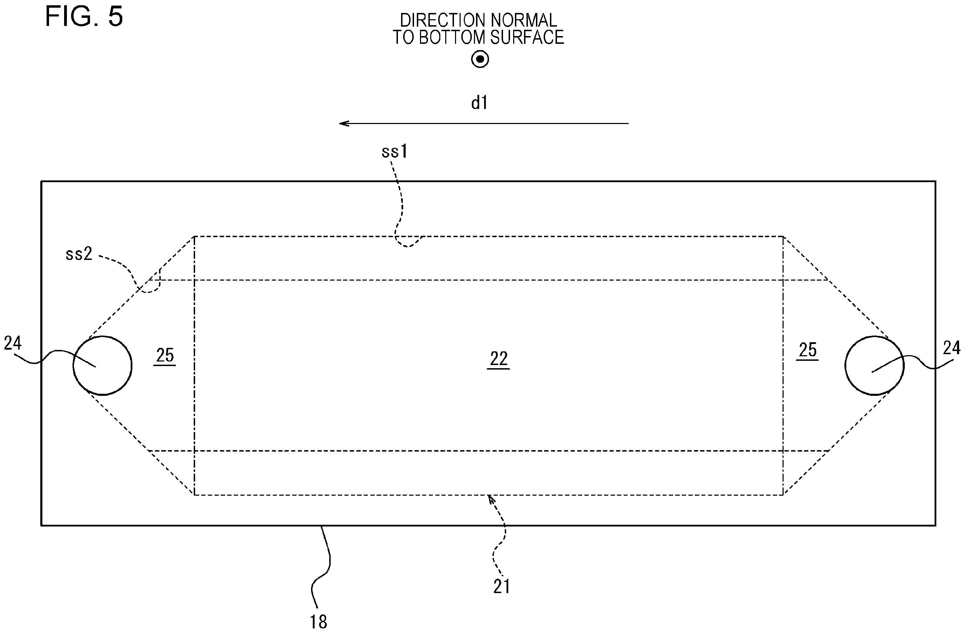

As illustrated in FIG. 5, the two ends of the internal flow path 21 in the first direction d1 may each have a shape that tapers with increasing distance from the center of the internal flow path 21 when viewed in a direction normal to the bottom surface bs. An inlet/outlet port 24 may be formed in the container 18 near the tip of each of the tapered shapes. The internal flow path 21 may have a tapered shape as described above as a result of the main part 22 being connected to inlet/outlet parts 25 at both ends of the main part 22 in the first direction d1.

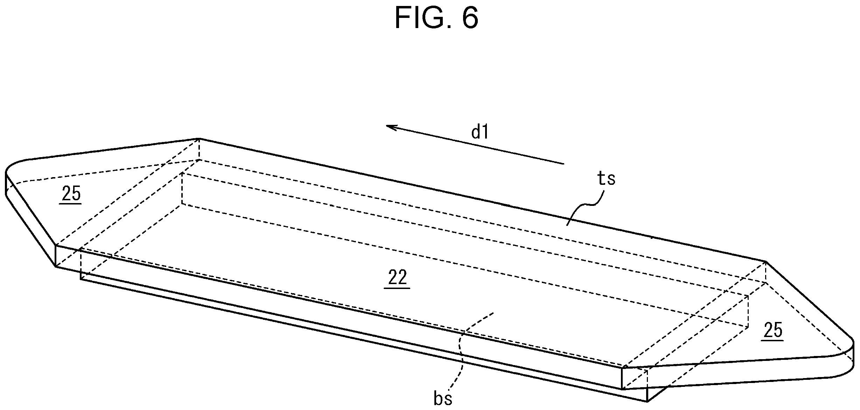

The inlet/outlet parts 25 may have the same bottom surface bs and top surface ts as the main part 22. Alternatively, as illustrated in FIG. 6, the inlet/outlet parts 25 may have the same top surface ts as the main part 22 but may have a bottom surface that is parallel to the bottom surface bs of the main part 22 and nearer the top surface ts. This bottom surface may be continuous with the surfaces of the step portions 23 facing the top surface ts. As illustrated in FIG. 5, the inlet/outlet parts 25 may have side surfaces ss2 that bend or curve inwardly in the second direction d2 from the side surfaces ss1 of the main part 22. The inlet/outlet parts 25 may be shaped to be symmetrical about an axis that is a straight line extending in the first direction d1 when viewed in a direction normal to the bottom surface bs. The inlet/outlet parts 25 may be substantially shaped like isosceles triangles that are connected to the main part 22 at their bottom edges when viewed in a direction normal to the bottom surface bs. In this embodiment, the inlet/outlet parts 25 are substantially right-angled isosceles triangles when viewed in a direction normal to the bottom surface bs.

The angle between the side surfaces ss2 of each of the inlet/outlet parts 25 may range from 60° to 120°. When the angle between the side surfaces ss2 of each of the inlet/outlet parts 25 is greater than or equal to 60°, an increase in the size of the sensor package 10 is avoided. When the angle between the side surfaces ss2 of each of the inlet/outlet parts 25 is less than or equal to 120°, fluid flowing into the internal flow path 21 can gradually spread in the second direction d2 while heading toward the main part 22 and this contributes to equalizing the flow velocity and internal pressure in the second direction d2.

As illustrated in FIG. 4, each of the inlet/outlet ports 24 may be defined by a cylindrical inner peripheral wall surface perpendicular to the bottom surface bs. The two inlet/outlet ports 24 may be located in the top surface ts. The inlet/outlet ports 24 communicate with the flow path 16 in the sensor module 11. The upper surface of a lid 27 containing the inlet/outlet ports 24 is flat, and therefore, for example, the lid 27 and the flow path 16 facing the lid 27 can be hermetically connected to each other via an O-ring. Therefore, the fluid can flow from the flow path 16 to the inlet/outlet ports 24 without leaking.

As illustrated in FIG. 2, the container 18 may be composed of a body 26 and the lid 27. The body 26 may include a cavity defined by the bottom surface bs and the two side surfaces ss1 of the main part 22 and the bottom surface bs and the two side surfaces ss2 of each inlet/outlet part 25. The inlet/outlet ports 24 may be formed in the lid 27. The cavity in the body 26 may be covered by the lid 27, thereby forming the internal flow path 21.

The container 18 may be made of a ceramic, a plastic, or a metal, for example. In this embodiment, when the container 18 is made of a ceramic, adsorption of the fluid and degassing from the container 18 can be suppressed.

The heater 20 may heat the internal flow path 21 and the sensors 19. The heater 20 may be disposed in the form of a layer inside the container 18. The heater 20 may be positioned on the side of the internal flow path 21 where the bottom surface bs is located. In this embodiment, the heater 20 is disposed in the form of a layer inside the body 26. The heater 20 is, for example, a ceramic heater.

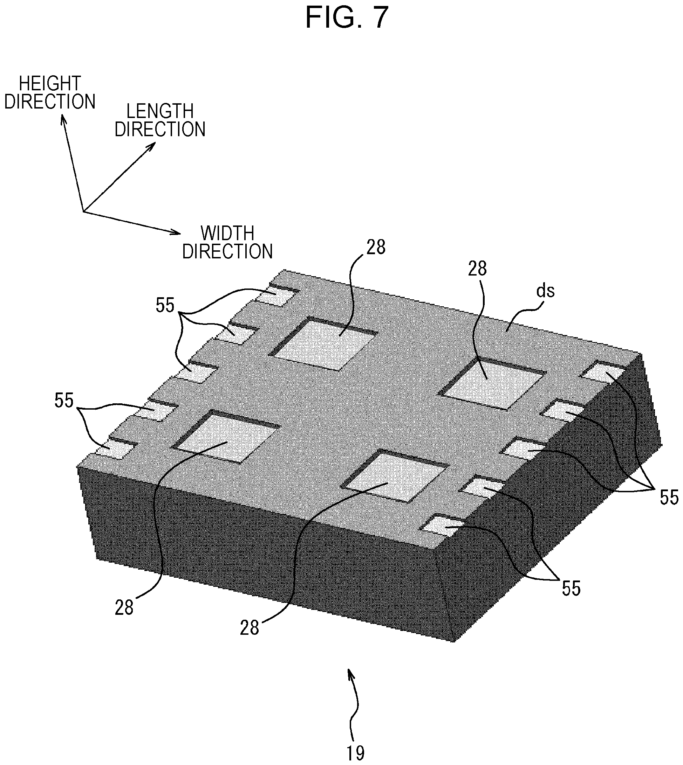

A length direction, a width direction, and a height direction may be defined in the sensors 19. As illustrated in FIG. 7, each sensor 19 may have a rectangular parallelepiped shape having flat surfaces that are each combinations of any two directions among a length direction, a width direction, and a height direction. Detection units 28 and sensor electrodes 55 may be provided on a surface of each sensor 19 on one side in the height direction. Hereinafter, the surface where the detection units 28 and the sensor electrodes 55 are provided is referred to as a detection surface ds. The sensor electrodes 55 may be located at the detection surface near at least one end or at both ends of the sensor 19 in the width direction. A plurality of detection units 28 may be provided and the detection units 28 may be disposed to be arrayed in the length direction and the width direction. In this embodiment, the sensor 19 is provided with four detection units 28 arrayed in the length direction and the width direction. The sensor 19 may have identical lengths in the length direction and the width direction. The plurality of sensors 19 may have identical sizes, i.e., identical lengths in the length direction, the width direction, and the height direction.

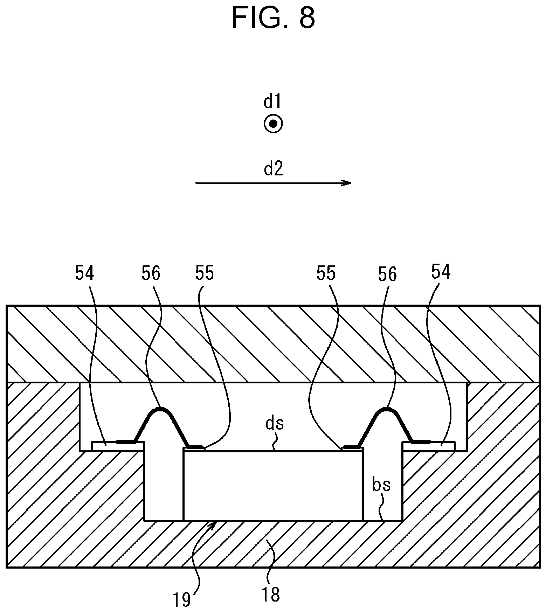

The plurality of sensors 19 is positioned and arrayed in the first direction d1 in the internal flow path 21 of the container 18. The plurality of sensors 19 may be fixed to be positioned on the bottom surface bs. As illustrated in FIG. 8, in this specification, "positioned on the bottom surface bs" means that the rear surface of each sensor 19, relative to the detection surface ds, contacts the bottom surface bs. The sensors 19 may be provided on the bottom surface bs while the length direction of the sensors 19 is parallel to the first direction d1 and the width direction of the sensors 19 is parallel to the second direction d2. The sensor electrodes 55 are connected, using connection wires 56, to step portion electrodes 54 located in the second direction relative to the sensor electrodes 55. The spacing between two sensors 19 that are adjacent to each other in the first direction d1 is preferably from 0.1 times to 1.0 times the length of the sensors 19. When the spacing is 0.1 times or more, residence of the fluid between the sensors 19 can be suppressed, the time taken for the fluid to be displaced inside the internal flow path 21 can be reduced, and mounting margin spaces can be secured for the sensors 19. When the spacing is 1.0 times or less, a reduction in the flow velocity is suppressed and the sensor package 10 avoids becoming unnecessarily large.

The detection units 28 have, for example, a film-like shape. The detection units 28 are particularly responsive to a certain component. At least one of the detection units 28 of the plurality of sensors 19 is particularly responsive to a first component, which is a component to be detected. In other words, at least one of the detection units 28 of the plurality of sensors 19 detects a component to be detected within the fluid. The detection units 28, for example, output a signal in response to adsorbing a particular component contained in the fluid. The detection units 28 are, for example, made of a polymeric material such as polystyrene, chloroprene rubber, polymethyl methacrylate or nitrocellulose, and a semiconductor material such as tin oxide or indium oxide. The detection units 28 output a signal in response to a particular component. This signal is, for example, output as a voltage value.

In FIG. 1, the measurement unit 14 includes a sensor capable of measuring predetermined properties or conditions related to the fluid supplied to the sensor module 11. The predetermined properties or conditions related to the fluid may be properties or conditions that may affect the accuracy of fluid detection in the sensor package 10. The predetermined properties or conditions related to the fluid may include the temperature or humidity of the fluid, for example. In this specification, the predetermined properties or conditions related to the fluid are the temperature and humidity of the fluid in the following description. In this case, the measurement unit 14 may include a temperature and humidity meter, for example. The temperature and humidity meter may measure the temperature and humidity of the fluid by use of a known existing method. The signals of the detection units 28 can be corrected by use of the temperature and humidity of the fluid measured by the measurement unit 14. However, the sensor module 11 does not necessarily include the measurement unit 14. The sensor module 11 can calculate the concentration of a component to be detected even without including the measurement unit 14.

The pump unit 15 draws the fluid supplied to the sensor module 11 from the upstream side into the downstream side and discharges the fluid to outside the sensor module 11. In other words, a fluid supplied to the sensor module 11 from the first flow path 17a or the second flow path 17b by suction performed by the pump unit 15 passes through the switching unit 13, the sensor package 10, the measurement unit 14, and the pump unit 15 and is then discharged to outside the sensor module 11 via the third flow path 17c. The pump unit 15 can control the rate at which the fluid is drawn in. For example, the flow velocity of the fluid flowing inside the flow path 16 is controlled by controlling the rate at which the fluid is drawn in by the pump unit 15. The pump unit 15 may, for example, control the rate at which the fluid is drawn in so as to suppress changes in the flow velocity of the fluid inside the flow path 16. The pump unit 15 may include a piezoelectric pump, for example. The pump unit 15 may include a single pump. The pump unit 15 may include a plurality of pumps. In this case, the plurality of pumps may be disposed in a line with respect to the flow of the fluid.

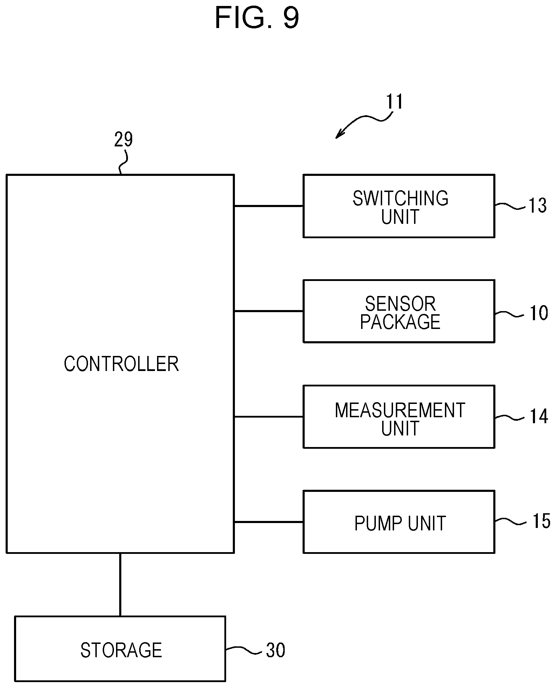

As illustrated in FIG. 9, the sensor module 11 may further include an electronic circuit board inside the housing 12. The electronic circuit board has a controller 29, a storage 30, and so forth of the sensor module 11, which are described later, mounted on the electronic circuit board.

FIG. 9 is a functional block diagram illustrating the schematic configuration of the sensor module 11 in FIG. 1. The sensor module 11 in FIG. 9 includes the controller 29, the storage 30, the switching unit 13, the sensor package 10, the measurement unit 14, and the pump unit 15.

The switching unit 13 receives a control signal from the controller 29 and switches between the first flow path 17a and the second flow path 17b based on the control signal. In this way, the test fluid or the control fluid is supplied to the flow path 16.

The sensor package 10 transmits and receives input and output signals of the sensors 19 to and from the controller 29.

The measurement unit 14 transmits and receives measured information signals to and from the controller 29.

The pump unit 15 receives a control signal from the controller 29. The pump unit 15 draws the fluid toward the downstream side based on the control signal. The pump unit 15 draws the fluid in at a rate in accordance with the control signal.

The controller 29 is a processor that controls and manages the entire sensor module 11 including the individual functional blocks of the sensor module 11. The controller 29 is composed of a processor such as a central processing unit (CPU) that executes a program stipulating control procedures. Such program is, for example, stored in the storage 30 or an external storage medium connected to the sensor module 11.

The controller 29 may calculate the concentration of a component to be detected within the test fluid based on a signal output from the sensor package 10. The controller 29 may also calculate the concentration of a component to be detected within the test fluid based on a signal output from the measurement unit 14. Depending on the properties or conditions of the fluid, the reactivity of the component to be detected in each sensor 19 of the sensor package 10 may vary. When the controller 29 calculates the concentration of a component to be detected in the test fluid based on a signal output from the measurement unit 14 in this way, the controller 29 can calculate the concentration of the component to be detected while reactivity is taken into consideration. Therefore, the accuracy with which the concentration of the component to be detected is calculated can be improved.

The storage 30 may include, for example, a semiconductor memory or a magnetic memory. The storage 30 stores, for example, various information and/or a program for causing the sensor module 11 to operate. The storage 30 may function as a work memory.

Control of the switching unit 13 and calculation of the concentration of a component to be detected performed by the controller 29 are described in detail.

A test fluid (sample gas) is supplied to the first flow path 17a. Here, as an example, a case in which the test fluid is the exhaled breath of a person is described. However the test fluid is not limited to being the exhaled breath of a person and may be any appropriate fluid that is to be tested. When the test fluid is the exhaled breath of a person, the component to be detected is, for example, acetone, ethanol, or carbon monoxide. The component to be detected is not limited to these examples. The test fluid contains a noise component (noise gas), which is a second component. The noise component is a component other than the component to be detected. All components other than the component to be detected such as oxygen, carbon dioxide, nitrogen, and water vapor are contained in the noise component.

The control fluid (refresh gas) is supplied to the second flow path 17b. The control fluid may, for example, be a fluid that substantially does not contain the component to be detected. Here, the meaning of "substantially does not contain the component to be detected" includes cases in which the component to be detected is not contained at all and cases in which the content of the component to be detected in the control fluid is extremely small compared to the content of the component to be detected in the test fluid to the extent of being considered practically nonexistent. When the test fluid is the exhaled breath of a person, for example, air can be used as the control fluid. However, the control fluid may be a fluid other than air. A noise component such as oxygen, carbon dioxide, nitrogen, and water vapor is contained in the control fluid.

The controller 29 maintains the draw-in rate of the pump unit 15 constant and switches the switching unit 13 between the first flow path 17a and the second flow path 17b at a constant time interval. The constant time interval may be set as appropriate in accordance with the type of test fluid or a property of the test fluid. Here, as an example, the constant time interval is 5 seconds. Therefore, the controller 29 controls the switching unit 13 to switch a flow path connected to the flow path 16 between the first flow path 17a and the second flow path 17b every 5 seconds.

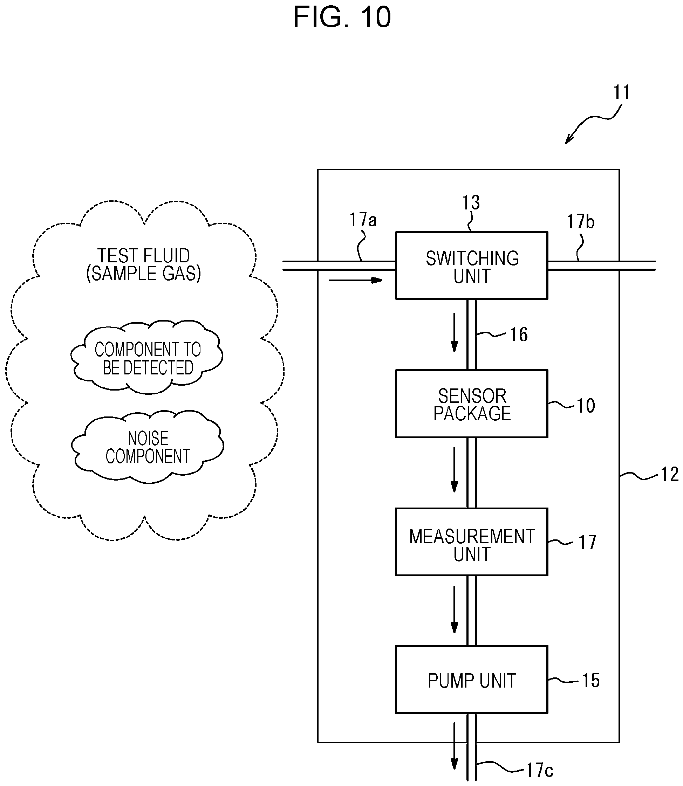

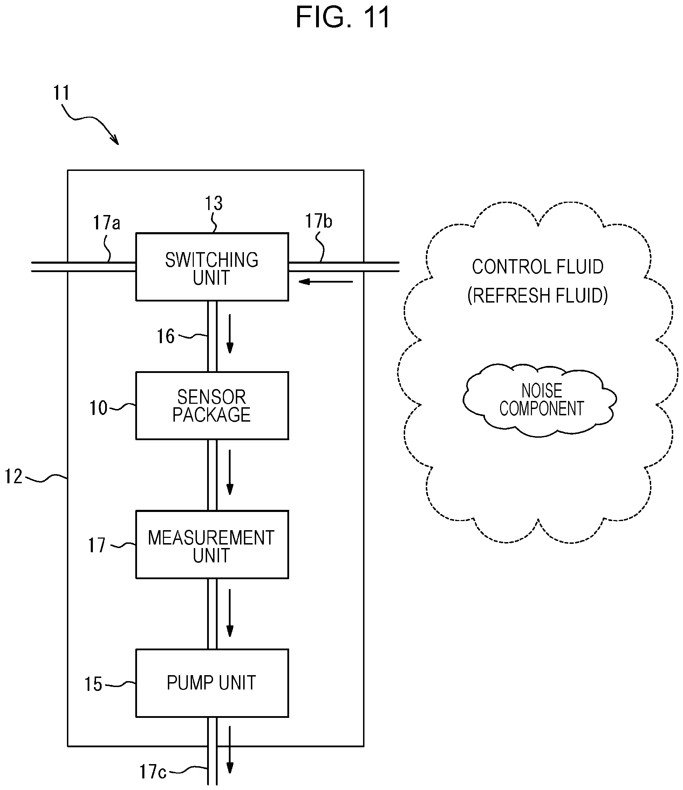

FIGs. 10 and 11 are diagrams schematically illustrating examples of fluid flow. FIG. 10 illustrates an example of a case in which the first flow path 17a is connected to the flow path 16. FIG. 11 illustrates an example of a case in which the second flow path 17b is connected to the flow path 16. In other words, in the example described here, the state in FIG. 10 and the state in FIG. 11 repeat in an alternating manner every 5 seconds. The arrows in FIGs. 10 and 11 indicate the direction of fluid flow.

As illustrated in FIG. 10, when the first flow path 17a is connected to the flow path 16, the test fluid is supplied to the sensor package 10 from the first flow path 17a by being drawn in by the pump unit 15. In this case, the detection units 28 of the sensors 19 of the sensor package 10 react to the components contained in the test fluid. Each sensor 19 outputs a signal (first signal) in accordance with components of the test fluid containing the component to be detected and the noise component.

As illustrated in FIG. 11, when the second flow path 17b is connected to the flow path 16, the control fluid is supplied to the sensor package 10 from the second flow path 17b by being drawn in by the pump unit 15. In this case, the sensors 19 of the sensor package 10 react to components contained in the control fluid. Each sensor 19 outputs a signal (second signal) in accordance with components of the control fluid containing the noise component.

The first signal and the second signal are signals that are supplied to the controller 29 in response to the sensor package 10 respectively reacting to the test fluid and the control fluid. The test fluid and the control fluid both contain the noise component. Therefore, the same responsiveness or similar degrees of responsiveness to the noise component in the fluids supplied to the sensor package 10 are reflected in both the first signal and the second signal.

In contrast, the test fluid contains the component to be detected, whereas the control fluid substantially does not contain the component to be detected. Therefore, the first signal is a signal that reflects responsiveness to the component to be detected, whereas the second signal is a signal that substantially does not reflect responsiveness to the component to be detected. Therefore, the difference between the first signal and the second signal output from the sensor package 10 can substantially represent the concentration of the component to be detected contained in the test fluid. The controller 29 can calculate the concentration of the component to be detected based on this difference.

In this embodiment, the thus-configured sensor package 10 includes the container 18 and the plurality of sensors 19. The container 18 includes the internal flow path 21 allowing a fluid to flow in the first direction d1. The plurality of sensors 19 is located in the internal flow path 21 and arrayed in the first direction d1. The plurality of sensors 19 detects the component to be detected in the fluid. With this configuration, the sensor package 10 is able to reduce overall fluid residence in the internal flow path 21 and reduce the residence time. Accordingly, the sensor package 10 can improve the responsiveness of the sensors 19 after a fluid has flowed into the sensor package 10. As a result of the improved responsiveness of the respective sensors 19, the sensor package 10 can detect odors resulting from a combination of components to be detected by each of the plurality of sensors 19 with high detection accuracy. Furthermore, in the sensor package 10 with the above-described configuration, since the pressure is equalized in the internal flow path 21, detection errors caused by differences in the pressure acting on the plurality of sensors 19 are reduced. Therefore, the sensor package 10 is able to realize further improved odor detection accuracy.

In the sensor package 10 of this embodiment, the plurality of sensors 19 is located on the flat bottom surface bs. With this configuration, in the sensor package 10, the bottom surface bs and the detection surfaces ds of the sensors 19 do not form a continuous plane but recessed steps are formed with respect to the detection surfaces ds. The fluid velocity between the detection surfaces ds and the top surface ts can be made homogeneous by these steps. This action has not been considered theoretically, but is inferred as follows. Fluids are viscous and therefore a fluid flowing along a surface that is entirely flat is thought to have a lower flow velocity in the vicinity of the surface. On the other hand, as in the above-described sensor package 10, a fluid flowing along a surface having steps that are recessed with respect to the detection surfaces ds is thought to have a flow velocity, decrease of which is suppressed by the recessed steps, near the surface where the detection units 28 are formed and the fluid flow velocity between the detection surfaces ds and the top surface ts can be made homogeneous.

In the sensor package 10 of this embodiment, the two ends of the internal flow path 21 in the first direction d1 are shaped so as to taper with increasing distance from the center of the internal flow path 21 when viewed in a direction normal to the bottom surface bs and the inlet/outlet port 24 is formed near the tip of each of the tapered shapes at both ends of the internal flow path 21 in the container 18. This configuration enables the sensor package 10 to equalize the pressure and the concentration of the fluid in the second direction d2 in the internal flow path 21. Therefore, the sensor package 10 is able to realize further improved odor detection accuracy.

In the sensor package 10 of this embodiment, the inlet/outlet ports 24 are defined by cylindrical inner peripheral wall surfaces that are perpendicular to the bottom surface bs. As a result of the sensor package 10 with this configuration, the fluid flowing into the internal flow path 21 from the inlet/outlet ports 24 can be made to hit the bottom surface bs and therefore the pressure can be equalized along the entire internal flow path 21. With this configuration, in the sensor package 10, the fluid easily flows in a space surrounded by the side surfaces of the sensors 19, the side surfaces of the step portions 23, and the bottom surface bs. With this configuration, in the sensor package 10, the fluid easily flows through spaces between the sensors 19 and surrounded by the bottom surface bs. As a result of these factors, the sensor package 10 can reduce residence of the fluid and can increase the flow velocity of the fluid in the first direction d1.

In the sensor package 10 of this embodiment, the container 18 includes the body 26 and the lid 27. The body 26 includes a cavity. The lid 27 contains the inlet/outlet ports 24. The internal flow path 21 is formed by the cavity being covered by the lid 27. With this configuration, the sensor package 10 can be manufactured by a simple method.

In the sensor package 10 of this embodiment, the container 18 is made of a ceramic. With this configuration, the sensor package 10 can reduce introduction of components of the container 18 into the fluid due to liquefaction or vaporization of the body of the container 18. Therefore, the sensor package 10 can suppress reduction of detection accuracy of the component to be detected.

In this embodiment, the sensor package 10 includes the heater 20. Therefore, in the sensor package 10, the heater 20 is used to heat the internal flow path 21 and the sensors 19. As a result, fluid adsorbed onto the internal flow path 21 and the sensors 19 desorb and in this way the internal flow path 21 can be refreshed. In the sensor package 10, variations in temperature inside the internal flow path 21 are reduced by the heater 20 and therefore a reduction in detection accuracy of the component to be detected can be suppressed regardless of changes in the temperature of the test fluid.

In the sensor package 10 of this embodiment, the step portions 23 extending in the first direction d1 are formed on both sides of the bottom surface bs in the second direction d2. With this configuration, the sensor package 10 collects more fluid between the detection surfaces ds of the sensors 19 and the top surface ts, increases the flow velocity, and reduces the fluid arrival time. In the sensor package 10, the connection wires 56 are disposed nearer the side surfaces ss1 in the second direction d2 than the detection units 28 and as a result the fluid can flow smoothly over the detection units 28 and the flow velocity of the fluid across the detection units 28 can be increased. On the other hand, the side surfaces ss1 are located away from the sensors 19 in the second direction d2 between the detection surfaces of the sensors 19 and the top surface ts in the sensor package 10 described above. Therefore, in the sensor package 10, reductions in the flow velocity of the fluid around edges in the second direction d2 between the surfaces where the detection units 28 are formed and the top surface ts are suppressed and differences in the flow velocity depending on differences in position in the second direction d2 can be reduced. If the entirety of each side surface ss1 is spaced away from the sensors 19, the flow velocity as a whole is reduced due to the increased volume of the internal flow path 21. On the other hand, with the above-described configuration, in the sensor package 10, since the entirety of each side surface ss1 is not spaced away from the sensors 19, differences in flow velocity can be reduced in regions that may contribute to improving detection accuracy while a reduction in the overall flow velocity being suppressed.

In the sensor package 10 of this embodiment, the height of the step portions 23 with respect to the bottom surface bs is greater than or equal to the height of the sensors 19. With this configuration, the sensor package 10 allows more fluid to flow over the sensors 19 and increases the flow velocity due to the space between the step portions 23 and the top surface ts made narrower. As a result, the sensor package 10 can reduce the residence time of the fluid and shorten differences between the detection times of the detection units 28 of the sensors 19, thereby having improved detection accuracy.

In the sensor package 10 of this embodiment, the sensor electrodes 55 and the step portion electrodes 54 are connected to each other by the connection wires 56 to allow signals output from the detection units 28 to be transmitted to outside the sensor package 10. This wiring structure suppresses reductions in the flow velocity of the fluid caused by connection wires and improves detection accuracy.

In this embodiment, the sensor module 11 draws in fluid using the pump unit 15 provided along the flow path 16 and supplies the fluid to the sensor package 10. The fluid supplied to the sensor package 10 is switched between the test fluid and the control fluid by the switch between the first flow path 17a and the second flow path 17b through the use of the switching unit 13. Therefore, a fluid is drawn in toward the downstream side using the same pump unit 15 regardless of whether the fluid supplied to the sensor package 10 is the test fluid or the control fluid. If different pumps are used to supply the test fluid and the control fluid to the sensor package 10, differences may occur between the amount of test fluid supplied and the amount of control fluid supplied due to differences in the performance of the pumps and so forth. However, in the sensor module 11 of this embodiment, since the fluid supplied to the sensor package 10 is controlled by the single pump unit 15, fluid can be supplied to the sensor package 10 more stably compared to a case where the different pumps are used to supply the fluids. This makes it easier to make the conditions under which the test fluid and the control fluid are supplied to the sensor package 10 identical. Therefore, the sensor package 10 is more likely to detect the test fluid and the control fluid under more equal conditions. Therefore, according to the sensor module 11, the accuracy with which a component to be detected is measured can be improved.

### EXAMPLES

Hereafter, the present disclosure is described in more detail by use of examples and a comparative example, but the present disclosure is not limited to these examples.

### [Steady Fluid Analysis and Transient Fluid Analysis]

Steady fluid analysis and transient fluid analysis were performed for examples and a comparative example described below. Ansys Fluent 19.2 (made by Ansys, Inc) was used in the steady fluid analysis and transient fluid analysis. The gas used in the steady fluid analysis and transient fluid analysis was air with a density of 1.225 kg/m³ (1 atmospheric pressure, 15 °C), a viscosity of 1.789 × 10⁻⁵ Pa ·s, and an inflow rate of 30 cm³/min.

### (Example 1)

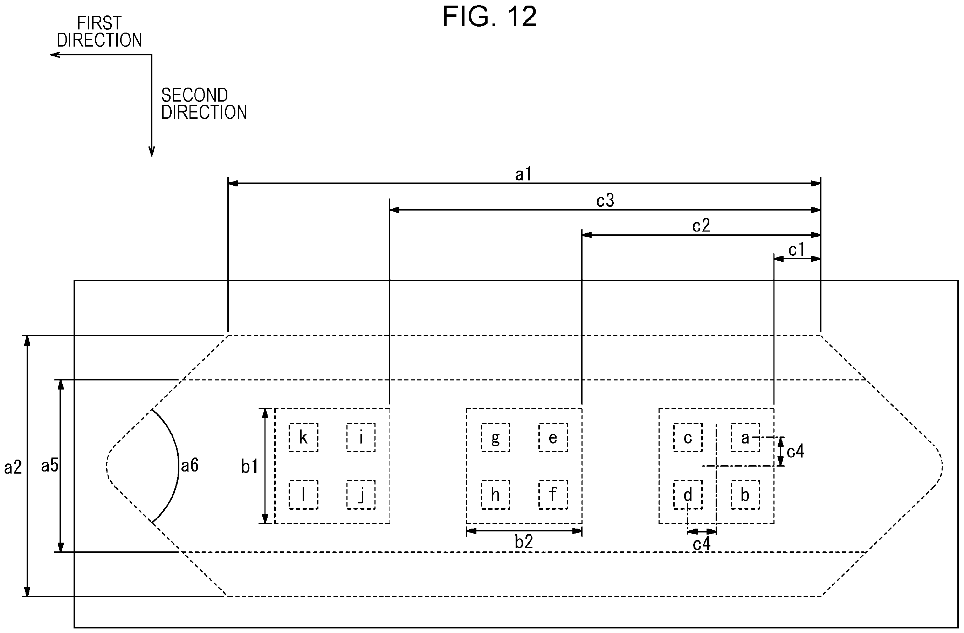

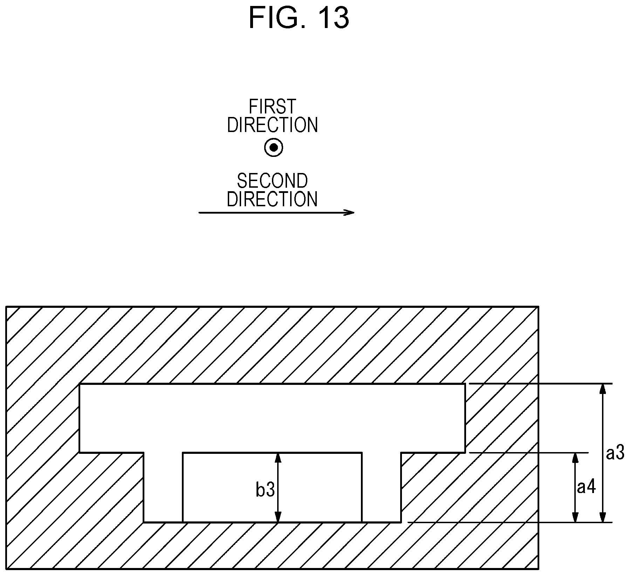

As illustrated in FIGs. 12 and 13, the internal flow path of Example 1 was modeled. A length a1 of the main part in the first direction was 9.20 mm. A spacing a2 between the side surfaces of the main part was 3.80 mm. A spacing a3 between the bottom surface and the top surface of the main part was 1.46 mm. A height a4 of the step portions from the bottom surface was 1.15 mm. A spacing a5 between the step portions formed on the two side surfaces was 2.70 mm. An angle a6 between the side surfaces of the inlet/outlet parts at both ends of the main part in the first direction was 90°. In this model, three rectangular parallelepiped shaped sensors were disposed on the bottom surface in the first direction inside the internal flow path of Example 1. The rectangular parallelepiped shaped sensors each had a length b1 of 2.1 mm, a width b2 of 2.1 mm, and a height b3 of 0.73 mm. In this model, each sensor was disposed so that the length direction and width direction of the sensor were respectively parallel to the first direction and the second direction of the internal flow path. In this model, the sensors were disposed in the first direction so that respective edges of the sensors were located at c1 = 0.46 mm, c2 = 3.56 mm, c3 = 6.66 mm from one end of the main part in the first direction. The detection units were disposed at positions shifted by c4 = 0.4 mm in the first direction and the second direction from the center of the corresponding sensor.

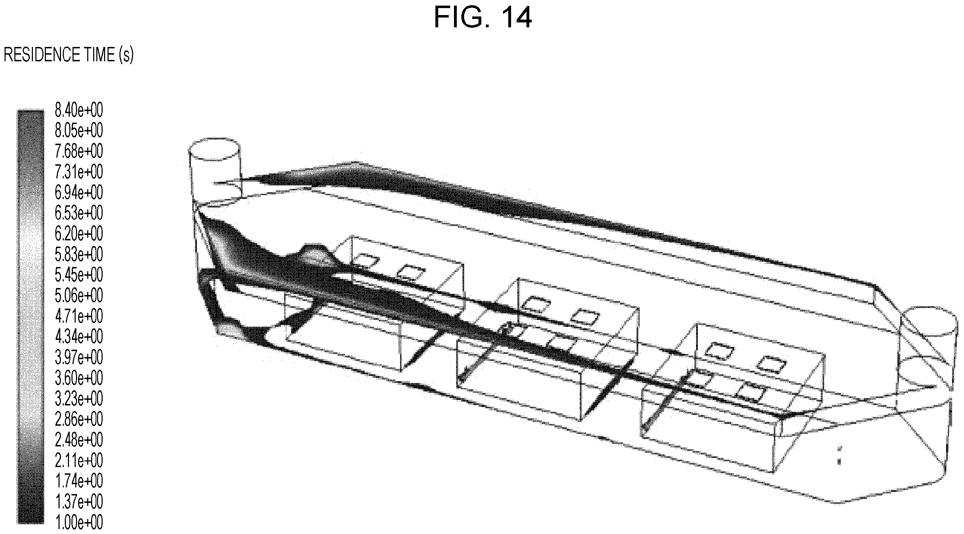

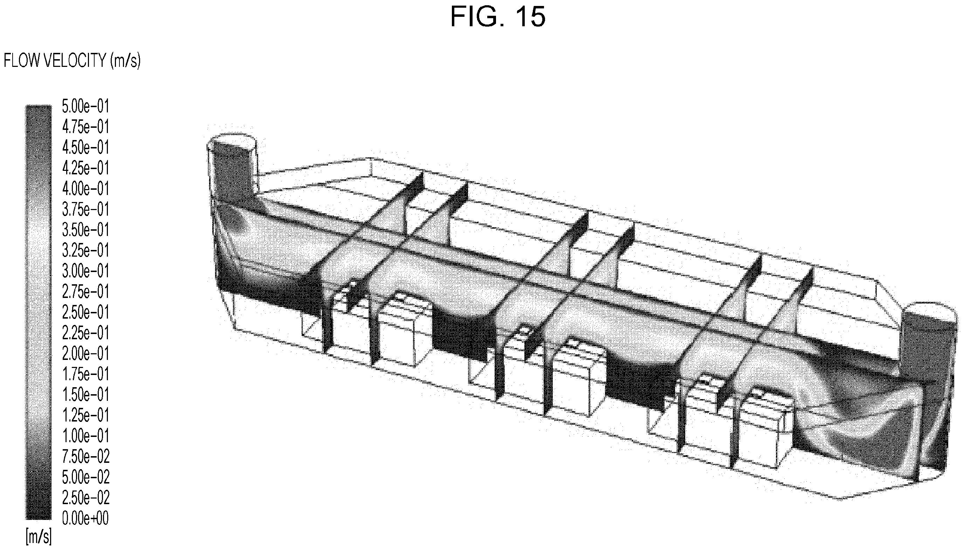

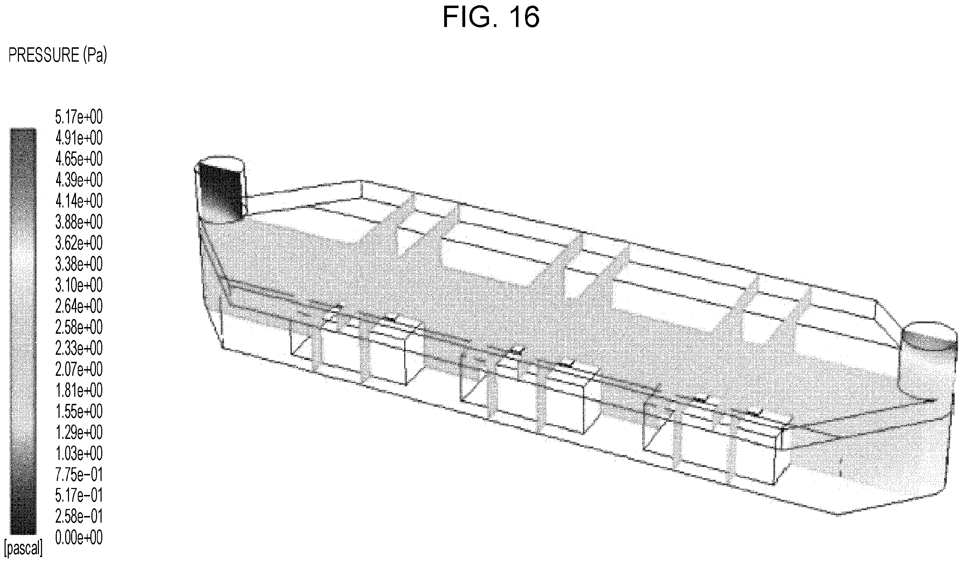

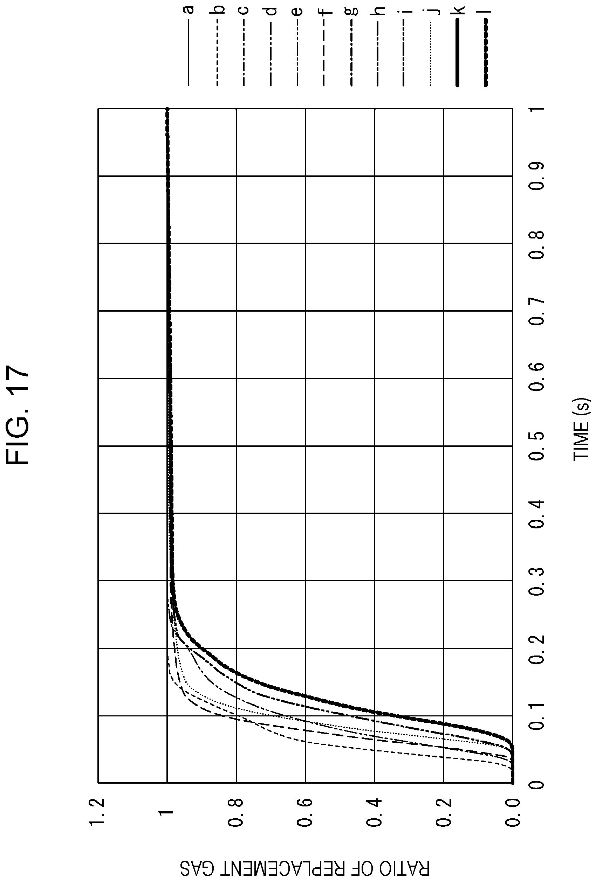

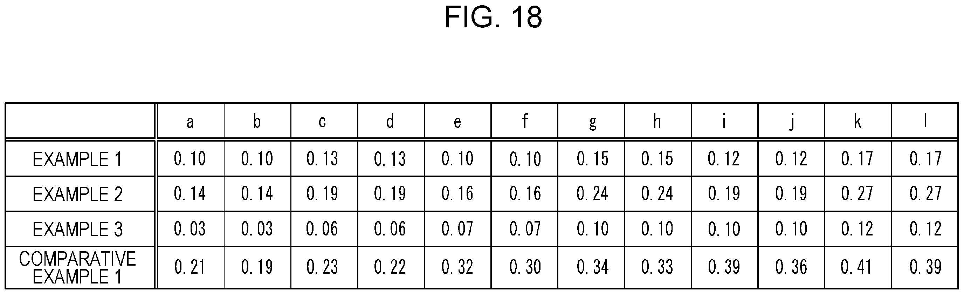

The residence time, flow velocity distribution, pressure distribution, and gas arrival time were calculated for the internal flow path of Example 1 for a case where gas was allowed to flow in from one outlet/inlet under the above-described conditions. FIG. 14 illustrates the residence time. In FIG. 14, residence times of less than 1 second are not illustrated. FIG. 15 illustrates the flow velocity distribution at a central position and at positions overlapping detection units in the second direction, and at positions overlapping the detection units in the first direction. FIG. 16 illustrates the pressure distribution at a central position and at positions overlapping the detection units in the second direction d2, and at positions overlapping the detection units in the first direction. FIG. 17 illustrates the ratio of replacement gas with respect to time after the start of inflow of gas at the positions indicated in FIG. 12 for detection units arrayed sequentially from the inflow side to the outflow side of the fluid. FIG. 18 illustrates the arrival times of the gas at the positions indicated in FIG. 12. In FIG. 18, the gas arrival time (s) is the time at which the ratio of replacement gas reaches 80% after the start of inflow of the gas.

### (Example 2)

As illustrated in FIG. 13, the internal flow path of Example 2 was modeled. The internal flow path of Example 2 was the same as the internal flow path of Example 1 except that the height a4 of the step portions from the bottom surface of the main part was 0.73 mm.

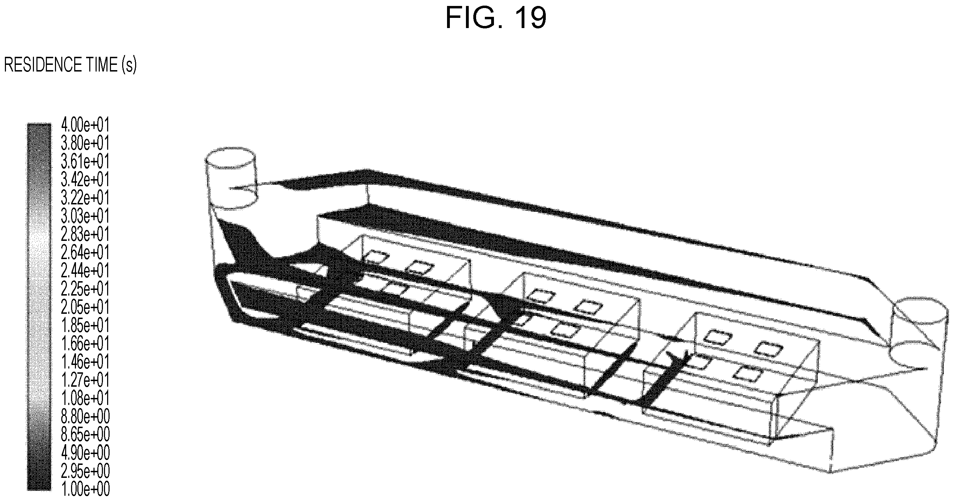

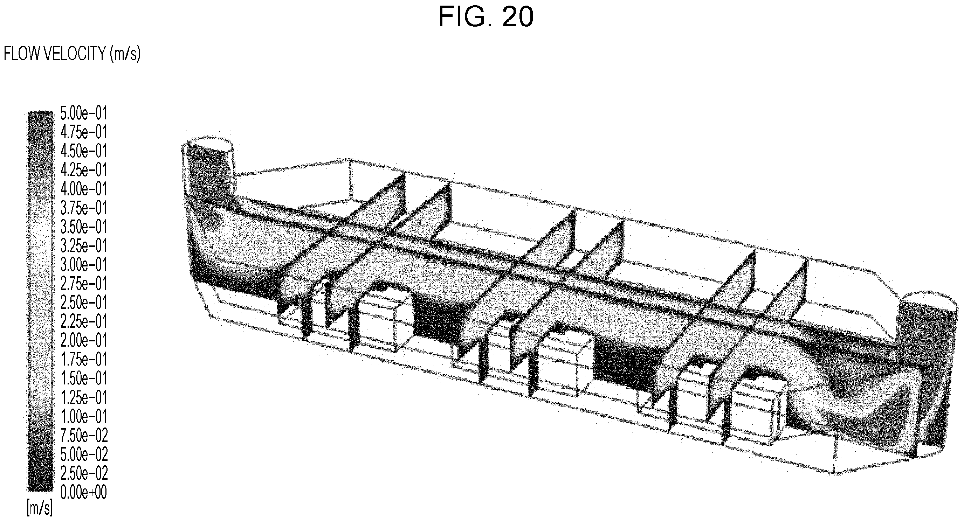

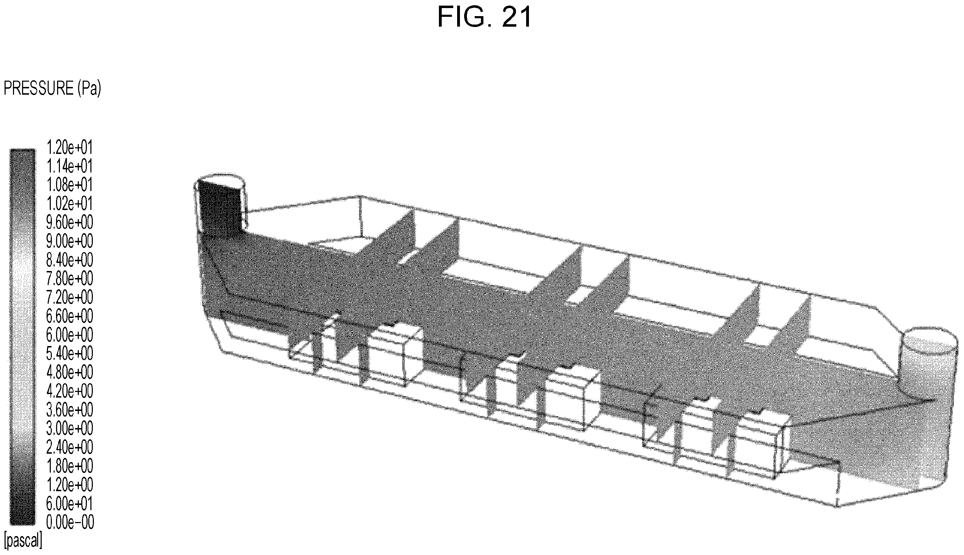

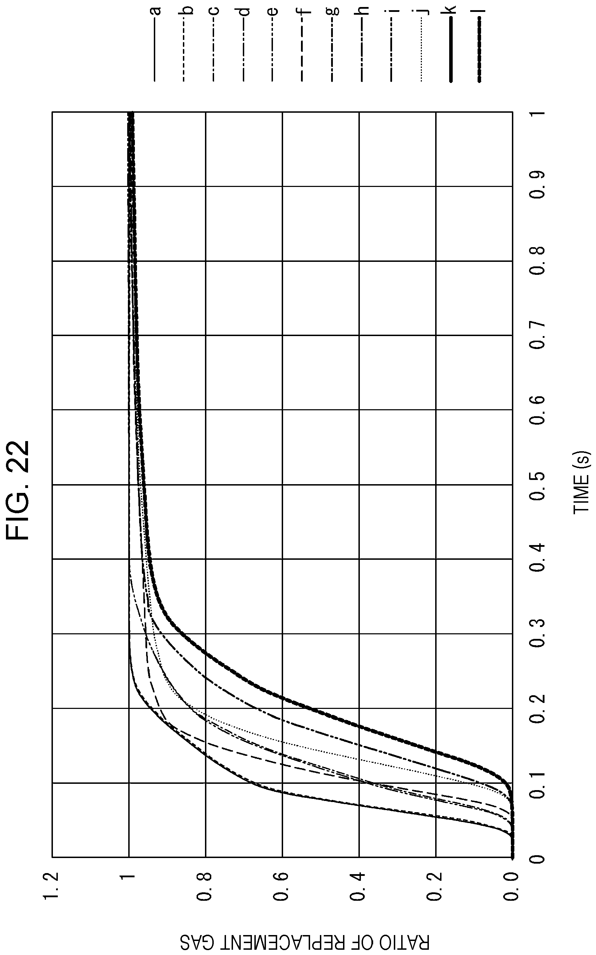

The residence time, flow velocity distribution, pressure distribution, and gas arrival time were calculated for the internal flow path of Example 2 for a case where gas was allowed to flow in from one outlet/inlet under the above-described conditions. FIG. 19 illustrates the residence time. In FIG. 19, residence times of less than 1 second are not illustrated. FIG. 20 illustrates the flow velocity distribution at a central position and at positions overlapping detection units in the second direction, and at positions overlapping the detection units in the first direction. FIG. 21 illustrates the pressure distribution at a central position and at positions overlapping the detection units in the second direction d2, and at positions overlapping the detection units in the first direction. FIG. 22 illustrates the ratio of replacement gas with respect to time after the start of inflow of gas at the positions indicated in FIG. 12 for detection units arrayed sequentially from the inflow side to the outflow side of the fluid. FIG. 18 illustrates the arrival times (s) of the gas at the positions indicated in FIG. 12.

### (Example 3)

Example 3 was modeled. The internal flow path of Example 3 was the same as that of Example 1 except that the spacing a3 between the bottom surface and the top surface of the main part was 1.10 mm and the height a4 of the step portions from the bottom surface was 0.73 mm, as illustrated in FIG. 13, and the bottom surfaces of inlet/outlet parts were continuous with the surfaces of the step portions facing the top surface of the container, as illustrated in FIG. 6.

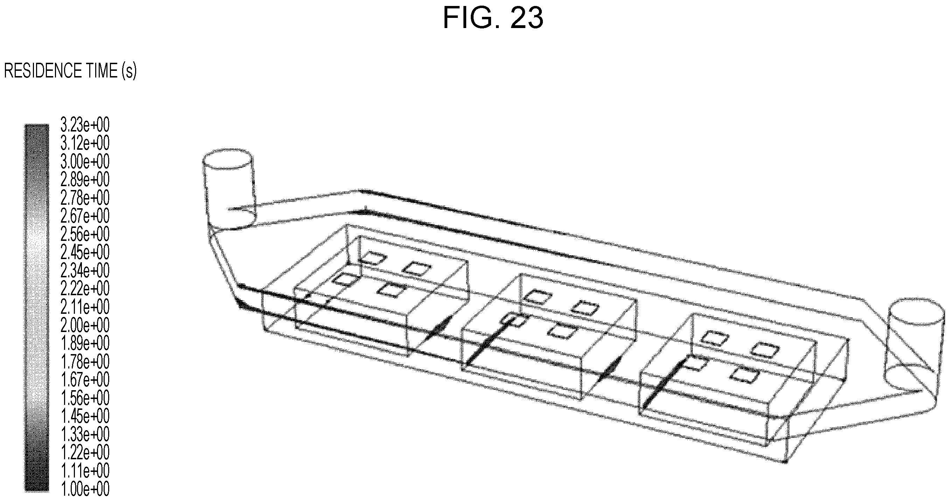

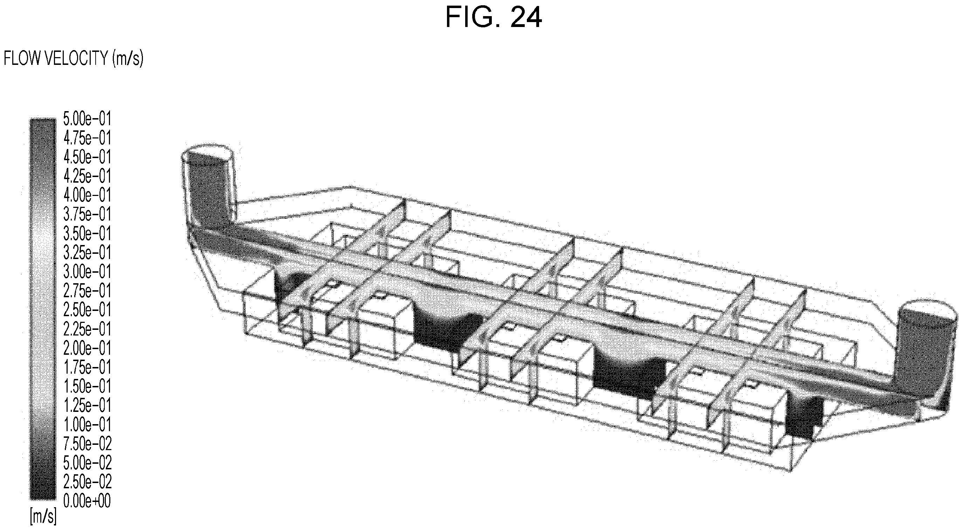

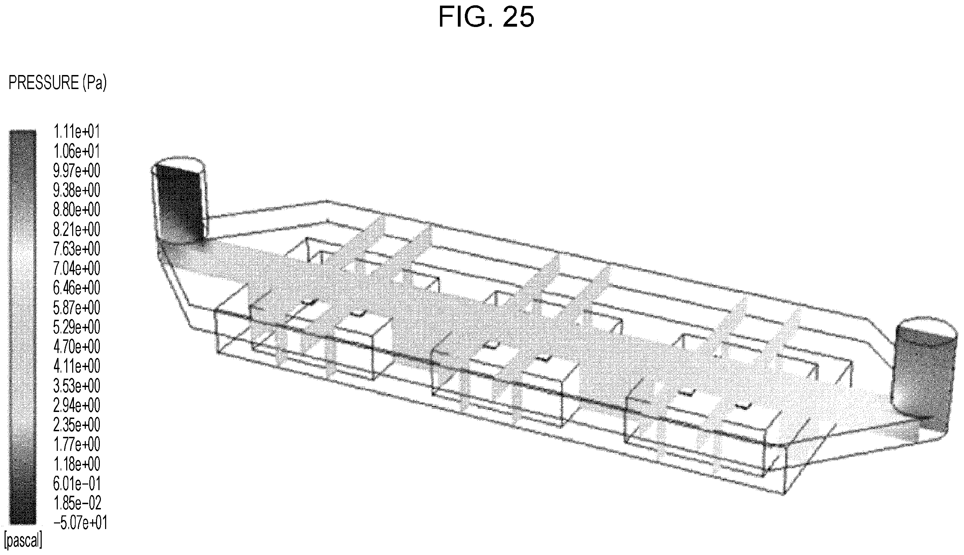

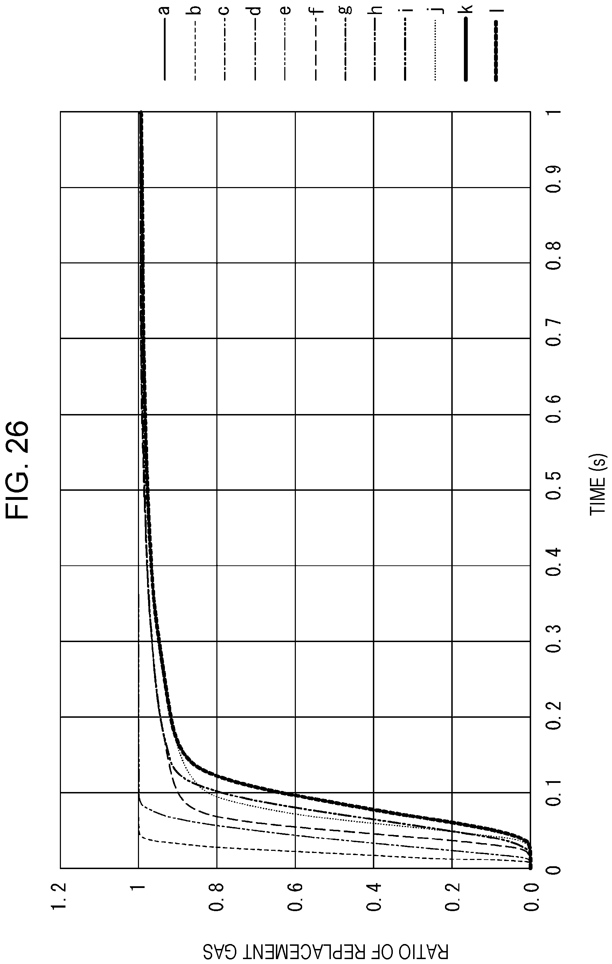

The residence time, flow velocity distribution, pressure distribution, and gas arrival time were calculated for the internal flow path of Example 3 for a case where gas was allowed to flow in from one outlet/inlet under the above-described conditions. FIG. 23 illustrates the residence time. In FIG. 23, residence times of less than 1 second are not illustrated. FIG. 24 illustrates the flow velocity distribution at a central position and at positions overlapping detection units in the second direction, and at positions overlapping the detection units in the first direction. FIG. 25 illustrates the pressure distribution at a central position and at positions overlapping the detection units in the second direction d2, and at positions overlapping the detection units in the first direction. FIG. 26 illustrates the ratio of replacement gas with respect to time after the start of inflow of gas at the positions indicated in FIG. 12 for detection units arrayed sequentially from the inflow side to the outflow side of the fluid. FIG. 18 illustrates the arrival times (s) of the gas at the positions indicated in FIG. 12.

### (Comparative Example 1)

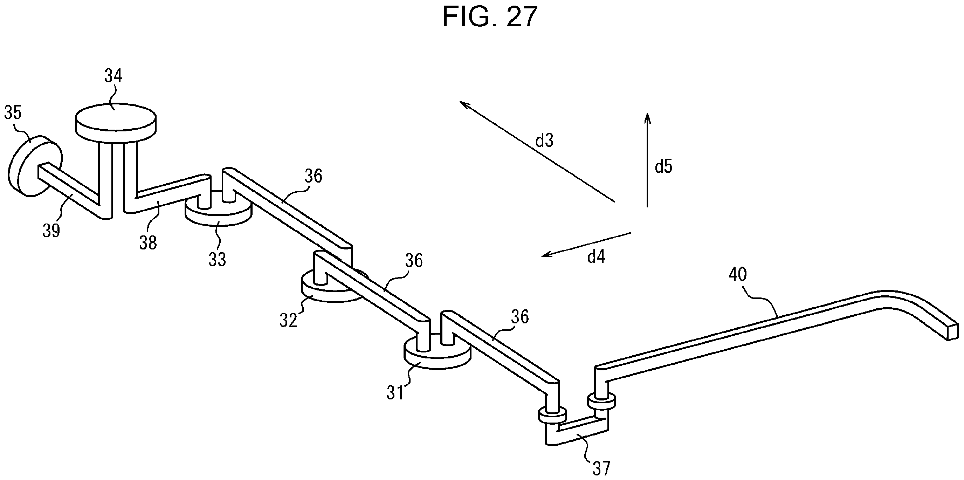

As illustrated in FIG. 27, a flow path of Comparative Example 1 was modeled by use of a first chamber 31, a second chamber 32, a third chamber 33, a fourth chamber 34, a fifth chamber 35, a first c-shaped connecting tube 36, a second c-shaped connecting tube 37, a z-shaped connecting tube 38, an l-shaped connecting tube 39, and a curved tube 40.

The first chamber 31, the second chamber 32, and the third chamber 33 were designed as cylinders having an inner diameter e1 of 3.0 mm and a height e2 of 0.7 mm. The fourth chamber 34 was designed as a cylinder having an inner diameter e3 of 4.0 mm and a height e4 of 0.7 mm. The fifth chamber 35 was designed as a cylinder having an inner diameter e5 of 4.2 mm and a height e6 of 1.0 mm.

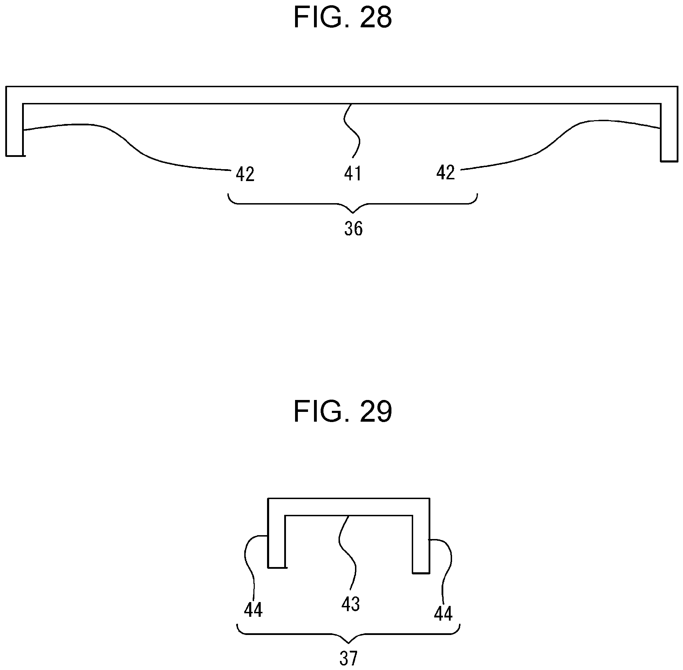

As illustrated in FIG. 28, the first c-shaped connecting tube 36 was designed to have a shape including a body portion 41 extending in a straight line and connecting portions 42 extending through identical lengths in the same direction perpendicular to a longitudinal direction of the body portion 41 from the two ends of the body portion 41 in the longitudinal direction. A length f1 of the body portion 41 was 10 mm. A length f2 of the connecting portions 42 was 2.5 mm. An inner diameter f3 of the first c-shaped connecting tube 36 was 1 mm. As illustrated in FIG. 29, the second c-shaped connecting tube 37 was designed to have a shape including a body portion 43 extending in a straight line and connecting portions 44 extending through identical lengths in the same direction perpendicular to a longitudinal direction of the body portion 43 from the two ends of the body portion 43 in the longitudinal direction. A length f4 of the body portion 43 was 4.2 mm. A length f5 of the connecting portions 44 was 2 mm. An inner diameter f3 of the second c-shaped connecting tube 37 was 1 mm.

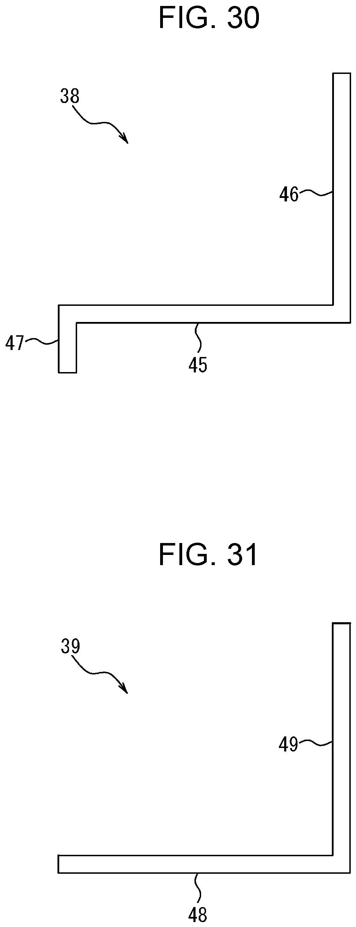

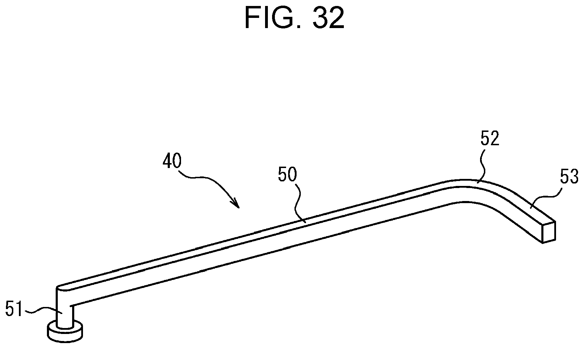

As illustrated in FIG. 30, the z-shaped connecting tube 38 was designed to have a shape including a body portion 45 extending in a straight line and a long connecting portion 46 and a short connecting portion 47 extending in opposite directions from each other perpendicular to a longitudinal direction of the body portion 45 at the two ends of the body portion 45 in the longitudinal direction. A length f6 of the body portion 45 was 7.5 mm. A length f7 of the long connecting portion 46 was 6.0 mm. A length f8 of the short connecting portion 47 was 2.5 mm. An inner diameter f3 of the z-shaped connecting tube 38 was 1.0 m. As illustrated in FIG. 31, the l-shaped connecting tube 39 was designed to have a shape including a body portion 48 extending in a straight line and a connecting portion 49 extending in a direction perpendicular to a longitudinal direction of the body portion 48 at one end of the body portion 48 in the longitudinal direction. A length f9 of the body portion 48 was 9.5 mm. A length f7 of the connecting portion 49 was 6.0 mm. An inner diameter f3 of the l-shaped connecting tube 39 was 1.0 mm. As illustrated in FIG. 32, the curved tube 40 was designed to have a shape including a body portion 50 extending in a straight line, a connecting portion 51 extending in a direction perpendicular to a longitudinal direction of the body portion 50 at one end of the body portion 50, a curved portion 52 curving in a direction perpendicular to the longitudinal direction at the other end, and an inlet portion 53 extending in a straight line from the other end of the bent portion 52. The bending direction of the connecting portion 51 with respect to the body portion 50 and the curving direction of the curved portion 52 are designed to be perpendicular to each other. A length f10 of the body portion 50 was 25 mm. A length f11 of the connecting portions 51 was 2.5 mm. A radius of curvature f12 of the curved portion 52 was 2.5 mm and the angle of curvature was 90°. A length f13 of the inlet portion 53 was 5.0 mm. An inner diameter f3 of the curved tube 4 was 1.0 mm.

As illustrated in FIG. 27, in the model of the flow path of Comparative Example 1, the first chamber 31, the second chamber 32, and the third chamber 33 were disposed sequentially in a third direction d3 with their bottom surfaces located on the same plane. In this model, the fourth chamber 34 was disposed so that the bottom surface of the fourth chamber 34 was parallel to the bottom surface of the third chamber 33 due to the fourth chamber being moved in a fourth direction d4 parallel to the bottom surface of the third chamber 33 and perpendicular to the third direction d3 and in a direction d5 normal to the bottom surface of the third chamber 33. In the model, the fifth chamber 35 was disposed so that the bottom surface of the fifth chamber 35 was perpendicular to the bottom surface of the fourth chamber 34 due to the fifth chamber 35 being moved, with respect to the fourth chamber 34, in the third direction with respect to the fourth chamber 34 and in a direction opposite to the direction d5 normal to the bottom surface of the third chamber 33.

In the model of the flow path of Comparative Example 1, the first chamber 31 and the second chamber 32 were connected to each other by the first c-shaped connecting tube 36 with the connecting portions 42 perpendicular to the bottom surface of the first chamber 31. In this model, the second chamber 32 and the third chamber 33 were connected to each other by the first c-shaped connecting tube 36 with the connecting portions 42 perpendicular to the bottom surface of the second chamber 32. In this model, the third chamber 33 and the fourth chamber 34 were connected to each other by the z-shaped connecting tube 38 with the long connecting portion 46 and the short connecting portion 47 perpendicular to the bottom surface of the third chamber 33. The short connecting portion 47 was connected to the third chamber 33. In this model, the fourth chamber 34 and the fifth chamber 35 were connected to each other by the l-shaped connecting tube 39 with the body portion 48 perpendicular to the bottom surface of the fifth chamber 35 and the connecting portion 49 perpendicular to the bottom surface of the fourth chamber 34. In this model, the first chamber 31 and the second c-shaped connecting tube 37 were connected to each other by the first c-shaped connecting tube 36 with the connecting portions 42 respectively perpendicular to the bottom surface of the first chamber 31 and in a straight line with the corresponding connecting portion 44 and with the connecting portions 42 extending in an opposite direction from the third direction d3. In this model, the connecting portion 51 of the curved tube 40 was connected in a straight continuous line to one connecting portion 44 of the second c-shaped connecting tube 37.

In the model of the flow path of Comparative Example 1, a sensor was disposed in the first chamber 31 such that the four detection units arrayed in the third direction d3 and the fourth direction d4 faced a position where the first chamber 31 was connected to the corresponding connecting portion 42 of the first c-shaped connecting tube 36. In this model, a sensor was disposed in the second chamber 32 such that the four detection units arrayed in the third direction d3 and the fourth direction d4 faced a position where the second chamber 32 was connected to the corresponding connecting portion 42 of the first c-shaped connecting tube 36. In this model, a sensor was disposed in the third chamber 33 such that the four detection units arrayed in the third direction d3 and the fourth direction d4 faced a position where the third chamber 33 was connected to the corresponding connecting portion 42 of the first c-shaped connecting tube 36.

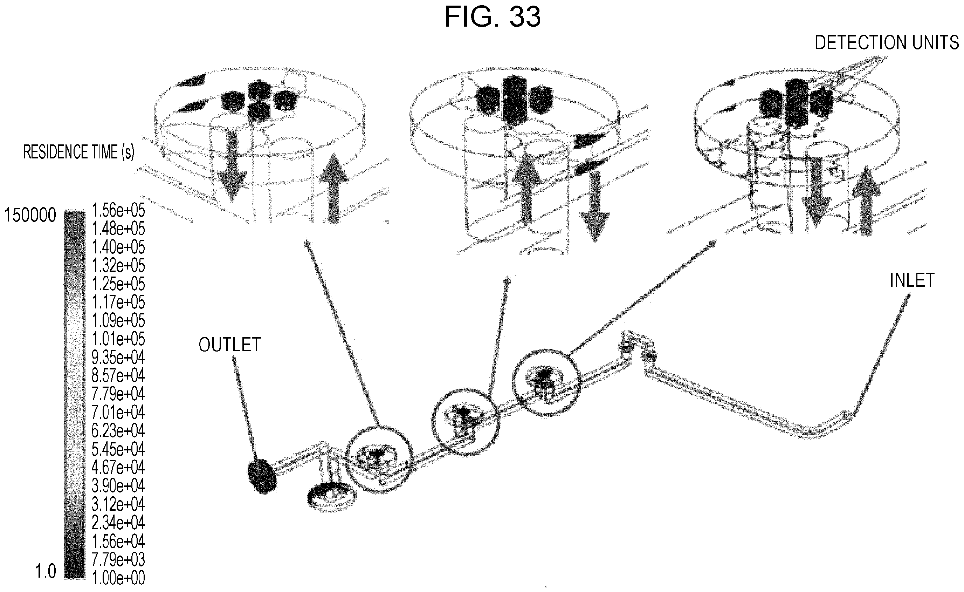

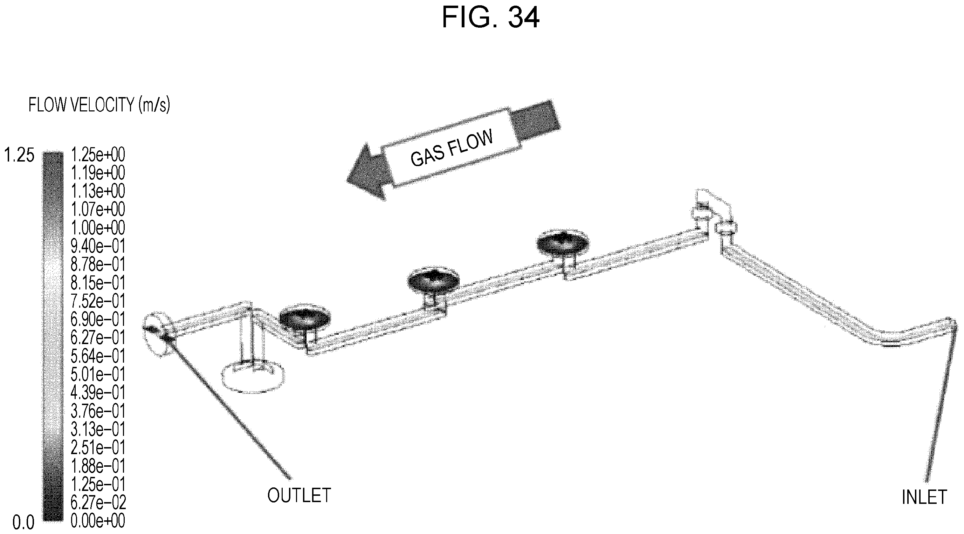

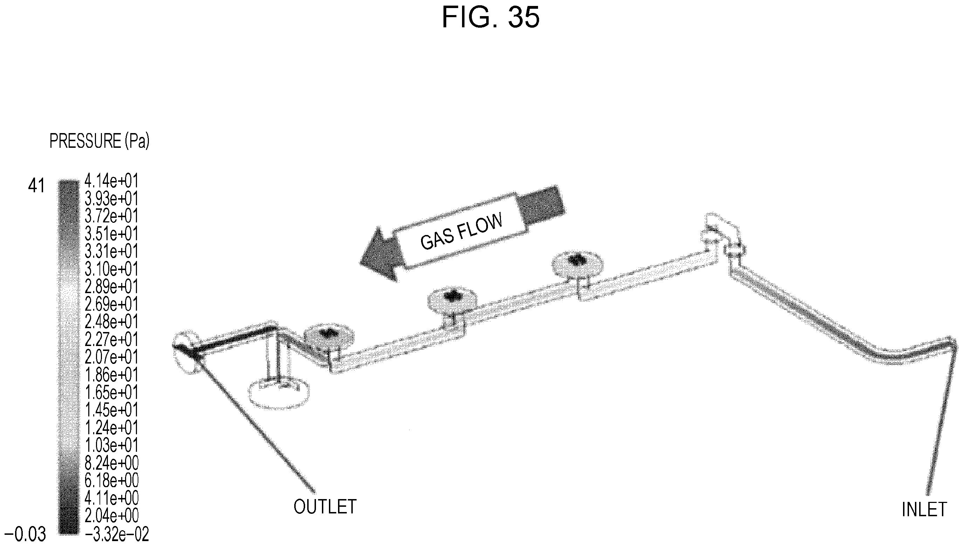

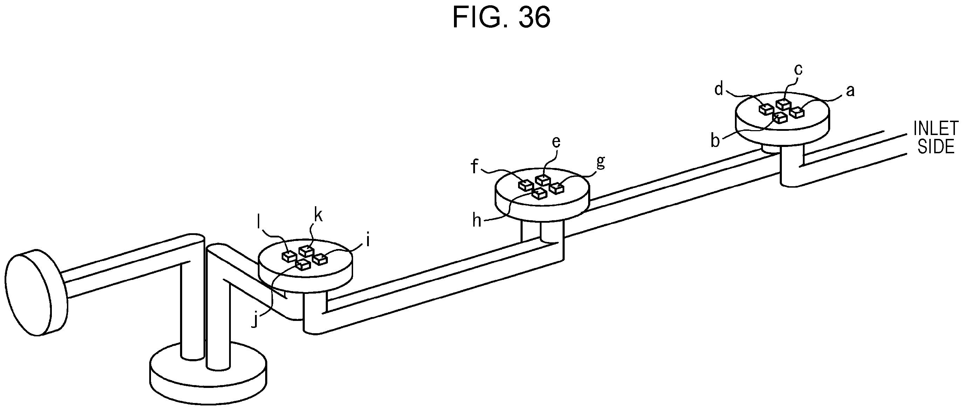

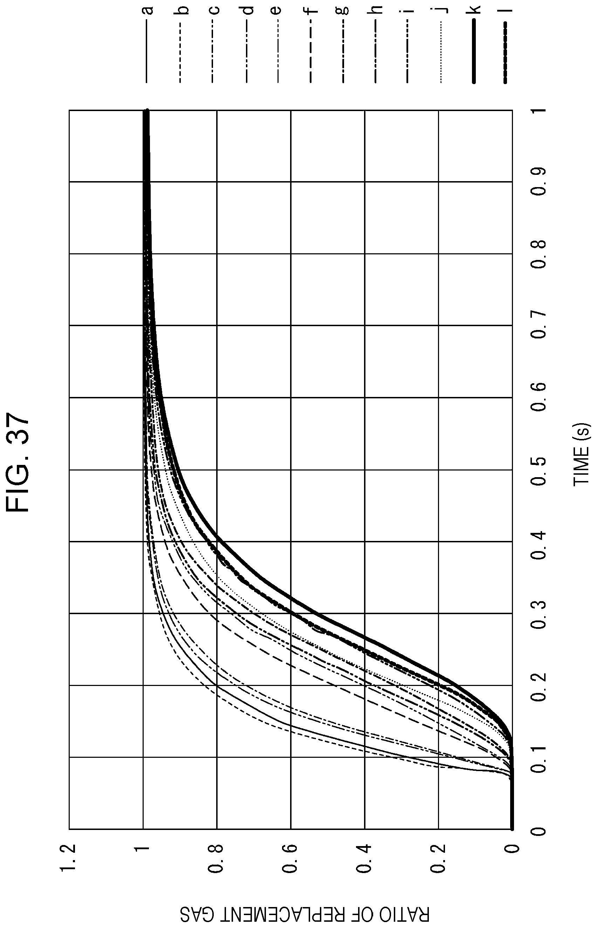

The residence time, flow velocity distribution, pressure distribution, and gas arrival time were calculated for the flow path of Comparative Example 1 for a case where gas was allowed to flow in from one outlet/inlet under the above-described conditions. FIG. 33 illustrates the residence time. In FIG. 33, residence times of less than 1 second are not illustrated. FIG. 34 illustrates the flow velocity distribution. FIG. 35 illustrates the pressure distribution. FIG. 37 illustrates the arrival times of the gas at the positions illustrated in FIG. 36 of detection units arrayed sequentially from the inflow side to the outflow side of the fluid.

The present disclosure has been described based on the drawings and examples. Note that a variety of variations and/or amendments may be easily made by one skilled in the art based on the present disclosure. Therefore, note that such variations and/or amendments are included within the scope of the present invention. For example, the functions included in each component can be rearranged in a logically consistent manner, and a plurality of components can be combined into a single component or a single component can be divided into a plurality of components.

Note that a system is disclosed herein as including various modules and/or units that perform specific functions. These modules and units are illustrated in a schematic manner to briefly illustrate their functionality and do not necessarily represent specific hardware and/or software. In that sense, these modules, units, and other components may be hardware and/or software implemented to substantially perform the specific functions described herein. The various functions of the different components may be any combination of hardware and/or software or hardware and/or software used in isolation, and can be used separately or in any combination. Input/output or I/O devices or user interfaces, including but not limited to keyboards, displays, touch screens, and pointing devices, can be connected directly to the system or via an I/O controller interposed between the system and those devices and interfaces. Thus, various aspects of the contents of the present disclosure can be implemented in numerous different ways, all of which are included within the scope of the present disclosure.

### REFERENCE SIGNS

- 10: sensor package
- 11: sensor module
- 12: housing
- 13: switching unit
- 14: measurement unit
- 15: pump unit
- 16: flow path
- 17a: first flow path
- 17b: second flow path
- 17c: third flow path
- 18: container
- 19: sensor
- 20: heater
- 21: internal flow path
- 22: main part
- 23: step portion
- 24: inlet/outlet port
- 25: inlet/outlet part
- 26: body
- 27: lid
- 28: detection unit
- 29: controller
- 30: storage
- 31: first chamber
- 32: second chamber
- 33: third chamber
- 34: fourth chamber
- 35: fifth chamber
- 36: first c-shaped connecting tube
- 37: second c-shaped connecting tube
- 38: z-shaped connecting tube
- 39: l-shaped connecting tube
- 40: curved tube
- 41: body portion
- 42: connecting portion
- 43: body portion
- 44: connecting portion
- 45: body portion
- 46: long connecting portion
- 47: short connecting portion
- 48: body portion
- 49: connecting portion
- 50: body portion
- 51: connecting portion
- 52: curved portion
- 53: inlet portion
- 54: step portion electrode
- 55: sensor electrode
- 56: connection wire
- bs: bottom surface
- d1: first direction
- d2: second direction
- d3: third direction
- d4: fourth direction
- d5: direction normal to bottom surface
- ds: detection surface
- ss1: side surface of main part
- ss2: side surface of inlet/outlet part
- s1: surface facing top surface
- ts: top surface
- w1: width of internal flow path
- w2: width of step portions formed on both side surfaces

## Claims

1. A sensor package comprising:
a container including inside an internal flow path allowing a fluid to flow in a first direction having linearity; and
a plurality of sensors located in the internal flow path and arrayed in the first direction, wherein the plurality of sensors is configured to detect a component to be detected within the fluid.

2. The sensor package according to claim 1,
wherein the plurality of sensors is located on a bottom surface being flat and defining part of the internal flow path.

3. The sensor package according to claim 2,
wherein two ends of the internal flow path in the first direction have tapered shapes that taper with increasing distance from a center of the internal flow path when viewed in a direction normal to the bottom surface, and
inlet/outlet ports are formed in the container near tips of the tapered shapes of the two ends of the internal flow path.

4. The sensor package according to claim 3,
wherein the inlet/outlet ports are defined by cylindrical inner peripheral wall surfaces perpendicular to the bottom surface.

5. The sensor package according to claim 3 or 4,
wherein the container includes a body including a cavity and a lid in which the inlet/outlet ports are formed, and
the internal flow path is formed by the cavity being covered by the lid.

6. The sensor package according to any one of claims 1 to 5,
wherein the container is made of a ceramic.

7. The sensor package according to any one of claims 1 to 6, further comprising:
a heater configured to heat the internal flow path.

8. The sensor package according to any one of claims 1 to 7,
wherein step portions extending in the first direction are formed at both sides of a bottom surface in a second direction, the second direction being parallel to the bottom surface partially defining the internal flow path and perpendicular to the first direction.

9. The sensor package according to claim 8,
wherein a height of the step portions with respect to the bottom surface is greater than or equal to a height of the plurality of sensors.

10. The sensor package according to claim 8 or 9,
wherein a width of the internal flow path in the second direction is from 1.5 times to 3 times a width of each of the plurality of sensors in the second direction.

11. The sensor package according to any one of claims 1 to 10,
wherein a spacing between each of the plurality of sensors and a top surface partially defining the internal flow path is equal to a height of each of the plurality of sensors.

12. A sensor module comprising:
a switching unit disposed downstream of a first flow path and a second flow path and configured to selectively switch open/closed states of the first flow path and the second flow path;
a sensor package disposed downstream of the switching unit and including a container including inside an internal flow path allowing a fluid to flow in a first direction having linearity and a plurality of sensors located in the internal flow path in the first direction and configured to detect a component to be detected within the fluid; and
a pump unit disposed downstream of the sensor package and configured to draw a fluid downstream.
